Europäisches Patentamt

European Patent Office

Office européen des brevets

(11) Veröffentlichungsnummer: **0 322 363 B1**

# EUROPÄISCHE PATENTSCHRIFT

(45) Veröffentlichungstag der Patentschrift: **30.12.92**

(51) Int. Cl.⁵: **A61B  17/14**, A61F 2/46

(21) Anmeldenummer: **88810867.7**

(22) Anmeldetag: **16.12.88**

(54) **Bezugssystem zur Implantation von kondylären Knie-Totalprothesen.**

(30) Priorität: **16.12.87 CH 4904/87**

(43) Veröffentlichungstag der Anmeldung:
**28.06.89 Patentblatt  89/26**

(45) Bekanntmachung des Hinweises auf die
Patenterteilung:
**30.12.92 Patentblatt  92/53**

(84) Benannte Vertragsstaaten:
**CH DE FR GB LI**

(56) Entgegenhaltungen:
**US-A- 3 670 724
US-A- 4 457 307
US-A- 4 524 766
US-A- 4 574 794**

(73) Patentinhaber: **Protek AG
Stadtbachstrasse 64
CH-3001 Bern(CH)**

(72) Erfinder: **Wehrli, Ulrich, Dr. med.
Gossetstrasse 29
CH-3084 Wabern/BE(CH)**

(74) Vertreter: **Fischer, Franz Josef
c/o Bovard AG Optingenstrasse 16
CH-3000 Bern 25(CH)**

# Beschreibung

Die vorliegende Erfindung betrifft ein Bezugssystem, welches es dem Chirurgen erlaubt, bei der Implantation von kondylären Knieprothesen die Osteotomien in äusserst präziser Weise durchzuführen. Dies ist eine Voraussetzung dafür, dass die Implantation so durchgeführt werden kann, dass durch die Operation die richtige mechanische Beinachse realisiert wird.

Es ist erwiesen, dass die Verwirklichung der richtigen mechanischen Beinachse eine der wichtigsten Voraussetzungen für eine lange Lebensdauer der Knieprothese ohne Lokkerung und Schmerz ist (Bargren, J.H., Blaha, J.D., Freeman, M.A.R.: Alignment in Total Knee Arthroplasty, Clinical Orthopaedics and Related Research 173: 178 - 183, 1983).

Dabei kommt es nicht nur darauf an, den richtigen statischen Knieaufbau (alignment) herzustellen, sondern es ist ebenfalls wichtig, dass die ausgewogene Spannung der Kniebänder, soweit sie erhalten sind bzw. die richtige Weichteilspannung vorliegt (F.C. Ewald: Biomechanical Indications for Implant Selection III: Knee. Role of Ligaments and Alignment, American Academy of Orthopaedic Surgeons, 54th Annual Meeting, San Francisco, January 22 - 27, 1987. Instructural Course Number 323).

Die kondylären Knieprothesen bestehen aus einer am Femur und einer an der Tibia befestigten Komponente, die im Gegensatz zur Scharnierprothese nicht durch künstliche mechanische Hilfsmittel miteinander verbunden sind.

Wie beim natürlichen Kniegelenk wird die flexible Verbindung zwischen den beiden Komponenten durch Bänder und Muskeln hergestellt, soweit diese bei der Implantation der Prothese erhalten bleiben. Bevor die Prothesenkomponenten auf dem Femur bzw. der Tibia durch Verklemmen, Verkeilen und/oder Knochenzement befestigt werden können, müssen Femur und Tibia mit Hilfe von Knochensägen und anderen Werkzeugen in die zur Prothese passende Form gebracht werden. Die im allgemeinen von den Herstellern der Knieprothese angebotenen Instrumente dienen in erster Linie dazu, die hierfür erforderlichen korrespondierenden Knochenschnitte am Femur und an der Tibia, die Osteotomien mit der notwendigen Genauigkeit vorzunehmen.

Dabei ist es eine wesentliche Forderung, dass die bei der Biegung und Streckung des Knies aneinandergleitenden Komponenten der Knieprothese immer die richtige Stellung zueinander haben, d.h. dass die mechanische Beinachse maximal 3° Varus oder 3° Valgus von der physiologischen Beinachse abweichen darf und dass eine Equilibrierung des Bandapparates erreicht wird, die sowohl bei der Extension als auch bei der Flexion eine gute Stabilität des Kniegelenks bewirkt.

Die bekannten Instrumentarien für die Implantation von kondylären Knie-Totalprothesen umfassen in der Regel folgende Mittel:
- Mittel zur Ausrichtung der Tibia auf dem Femur zur Erzielung der gewünschten Beinachsenstellung.
- Mittel zur Durchführung von Osteotomien, in Form von Schneidlehren, welche der Führung der Säge dienen. Diese Schneidlehren sind auf bestimmte Prothesenformen abgestimmt und lassen sich meist für verschiedene Grössen eines Prothesentyps verwenden.
- Mittel zur Herstellung der gewünschten Spannung der Kniebänder.

Ein solches Instrumentarium ist in der US-A-4 524 766 beschrieben. Es umfasst eine Führungsvorrichtung, welche frontal an der Tibia befestigt wird. Zur Durchführung der Osteotomien ist am oberen Ende dieser Führungsvorrichtung ein Schneidblock einstellbar angeordnet. Diese Anordnung hat jedoch den Nachteil, dass durch den oberen Teil der Führungsvorrichtung angrenzend an den Schneidblock der Zugang zum Operationsfeld beeinträchtigt wird. Im weiteren ist eine präzise Ausrichtung des Knieaufbaus schwierig, da der Bezugspunkt der Operation am Beckenknochen auf den Operationstisch ausgerichtet ist.

Weitere Instrumentarien sind beispielsweise in den folgenden Druckschriften beschrieben: Richards GmbH, Osterbrooksweg 69, 2000 Hamburg-Schenefeld: TRICON-M (R) "Die zementfreie Kniegelenksendoprothese", Operationstechnik mit dem ProFit TM Instrumentarium, ohne Datum. Pappas, M.J., Buechel, F.F.: N.J. Knee Instrumentation System: Biomechanical and Surgical Rationale, Biomedical Engineering Corp., Issue Date 7/1984. ZIMMER (R) Intramedullary Surgical Technique for the Miller/Galante Total Knee System, ohne Datum. Intermedics Orthopaedics, 1300 East Anderson Lane, Austin, Texas 78752: The Intermedics Natural-Knee TM System, 1986. Howmedica, Inc., Orthopaedics Division, 359 Veterans Blvd., Rutherford, N.J. 07070: The Howmedica (R) Total Knee Instrument System, 1980. Interplanta GmbH, Barkhausenweg 10, 2000 Hamburg 63: Schalen-Kniegelenkprothesen-System Modell Interplanta (C), W. Link, 1981.

Die bekannten Instrumentarien weisen den Nachteil auf, dass die Schneidlehre für die Osteotomien zwei Bezugssysteme besitzt, nämlich eines an der Tibia für Schnitte an derselben und ein zweites am Femur für Schnitte am Femur. Zur Bestimmung der Schnittebenen und zur Ausführung der Osteotomie mit der Säge an der Tibia werden Messmittel und Schneidlehre zunächst an der Tibia, z.B. mit Steinmann-Nägeln oder Knochenschrauben befestigt. Nach der Ausführung der Tibia-Schnitte werden Messmittel und Schneidlehre am Femur befestigt. Durch diesen Wechsel ist eine genaue geometrische Beziehung zwischen der Tibia-seitigen und der Femur-seitigen Gelenkprothesenkomponente nicht gewährleistet. Tatsächlich sind diesbezügliche Fehler jedem Chirurgen, der kondyläre Knie-Totalprothesen implantiert, bekannt. Solche Fehler sind beispielsweise beschrieben von Lotke, P.A., Ecker, M.L.: Influence of Positioning of Prosthesis in Total Knee Replacement, J. of Bone and Joint Surgery 55-A, 1977, 77 - 79.

Es wurden auch schon Massnahmen getroffen, um den genannten Nachteil zu vermeiden, beispielsweise indem das Knie mittels einer mechanischen Vorrichtung am Operationstisch befestigt wurde, wobei auch die Säge mit dieser Vorrichtung verbunden war (siehe Cooke, T.D.V., Saunders, G., Siu, D., Yoshioka, Y., Wevers, W.: Application of bench mounted saws for precision arthroplasty of the arthritic knee (Questor knee jigs). Abstract Book, Second European Congress of Knee Surgery and Arthroscopy, Basel, September 29 - October 4, 1986, p. 110 - 111. J. Biomed. Eng. 7: 45 - 50, 1985).

Die in den genannten Publikationen beschriebenen Vorrichtungen umfassen aufwendige mechanische Konstruktionen, deren Anwendung zeitaufwendig ist und ebenfalls den Zugang zum Operationsfeld behindern.

Es ist demzufolge Aufgabe der vorliegenden Erfindung, ein Instrumentarium zu schaffen, welches die obenbeschriebenen Nachteile der bekannten Instrumentarien vermeidet.

Es wurde gefunden, dass diese Aufgabe gelöst werden konnte, indem ein Bezugssystem zur Implantation von kondylären Knie-Totalprothesen geschaffen wurde, in welchem die Messmittel und die Schneidlehre für die tibialen und femoralen Osteotomien allein an der Tibia befestigt sind. Das Bezugssystem muss so ausgebildet sein, dass es während der gesamten Operation an der Tibia angeschraubt bleiben kann, ohne dass der Zugang zum Operationsfeld wesentlich behindert wird und ohne dass Bewegungen des Kniegelenkes verhindert werden.

Gegenstand der vorliegenden Erfindung ist demzufolge das in Patentanspruch 1 definierte Bezugssystem zur Implantation von kondylären Knie-Totalprothesen.

Das erfindungsgemässe Bezugssystem stellt ein Instrumentarium dar, mit welchem die Schneidlehre für die tibialen und femoralen Osteotomien vom gleichen Bezugspunkt aus eingestellt werden kann. Das Instrumentarium ist dabei vorzugsweise mittels zwei Befestigungsarmen seitlich an der Tibia mittels modifizierten Schanzschrauben befestigt. Das Basiselement des Instrumentariums ist der Messstab, welcher mittels der erwähnten Befestigungsarme vorzugsweise etwa 10 cm von der Tibiaachse entfernt befestigt wird. An diesem Messstab ist der Schneidblock auf der Führungsschiene beweglich angebracht, welcher präzise vor dem eröffneten Knie in Position gebracht werden kann. Am genannten Messstab ist ebenfalls ein sogenannter Zielstab vorhanden, welcher im wesentlichen die Verlängerung des Messstabs darstellt und bis in die Gegend des Beckenknochens reicht. Vor der Operation wird ein Zielelement, welches oben am Zielstab verschiebbar angeordnet ist bezüglich des Zielstabs z.B. mit einer Schraube so fixiert, dass es auf einen bestimmten Punkt am Beckenknochen zeigt. Dieser Punkt ist vorzugsweise eine in die Spina iliaca anterior superior senkrecht zur Frontalebene eingeschraubte Schraube. Durch das erfindungsgemässe Bezugssystem kann auch während der Operation die Tibia bezüglich dem Femur jederzeit in die richtige Position gebracht werden. Dies ist insbesondere dann erforderlich, wenn Schnitte am Femur durchgeführt werden müssen und in einem späten Stadium der Operation zum richtigen Einsetzen der kondylären Knie-Totalprothese.

Der Zielstab weist vorzugsweise an der Stelle, wo er mit dem Messstab verbunden ist, ein Gelenk auf, welches das Wegklappen erlaubt, damit die gute Zugänglichkeit des Operationsfeldes gewährleistet bleibt. Für die Osteotomie der ventralen und dorsalen Femurkondylen wird das Knie in flektierte Lage gebracht. Auch diese Position ist bei der Verwendung des erfindungsgemässen Bezugssystem möglich. Wird in der ersten Phase der Operation die Osteotomie der ventralen und dorsalen Femurkondylen, in der zweiten Phase die Osteotomie der Tibia und in der dritten Phase die Osteotomie der distalen Kondylen durchgeführt, wird vor der Durchführung dieser zweiten und dritten Phase zwischen die Tibia und das Femur ein Spanninstrument eingesetzt. In diesem Zustand ziehen die gespannten Bänder und Gewebe die beiden Knochen zusammen.

Deshalb sind Hilfsmittel erforderlich, um die Tibia in die richtige Position zu bringen, damit die Osteotomien bei 90° flektiertem Gelenk an Tibia und Femur und bei gestrecktem Bein am Femur in korrekter Weise durchgeführt werden können. Ein solches Hilfsmittel ist das Spanninstrument. Es hat die Aufgabe, die Gelenkflächen der Tibia und des Femur auseinanderzudrücken, wobei dieser Vorgang durch das erfindungsgemässe Bezugssystem kontrolliert werden kann.

Nachstehend sind zwei verschiedene Beispiele von Spanninstrumenten beschrieben, das eine für die Osteotomien am gestreckten, das andere für Osteotomien am flektierten Knie.

Das erste Spanninstrument für die Osteotomien am gestreckten Knie ist vorzugsweise eine blockförmige Vorrichtung, die zwischen Femur und Tibia eingeführt werden kann, nachdem die Osteotomie des Tibiakopfes durchgeführt worden ist. Der Block ist vorzugsweise so ausgestaltet, dass auf seiner Oberseite zwei bewegliche Platten unabhängig voneinander parallel zu einer auf der Tibia aufliegenden Platte beispielsweise durch Anziehen von Schrauben in Richtung Femur verschoben werden können, so dass verschiedene Abstände zwischen der medialen und lateralen Femurkondyle eingestellt und verschiedene Kräfte ausgeübt werden können. Auf diese Weise ist es möglich, die Weichteilspannung medial und lateral richtig einzustellen und eine eventuell auszuführende Achsenkorrektur zu prüfen.

Das zweite Spanninstrument ist nach dem Prinzip eine Spreizzange konstruiert und weist in Verbindung mit dem erfindungsgemässen Bezugssystem gegenüber bekannten derartigen Instrumenten eine Reihe von Vorteilen auf. Das Spanninstrument kann ohne Entfernung des Schneidblockes bei flektiertem Knie eingesetzt werden. Dabei kann ein Arm des Spanninstruments durch eine Oeffnung so eingeführt werden, dass es am Dach der Fossa intercondylica anliegt. Der andere Arm des Spanninstruments ist so ausgebildet, dass er sich auf einer unterhalb des Knies in die Tibia eingedrehten Knochenschraube abstützt. Diese Knochenschraube, eine modifizierte Schanzschraube, ist bereits zur Befestigung des knieseitigen Befestigungsarmes eingedreht worden. Die Abstützung an dieser Schraube hat gegenüber der Abstützung auf der dem Femur benachbarten Tibiaosteotomie den Vorteil, dass die Osteotomiefläche durch die Krafteinwirkung nicht beschädigt wird. Das Spanninstrument hat ferner den Vorteil, dass es durch die von den medial und lateral unterschiedlichen Weichteilkräften verursachte Rotation des Kniegelenks nicht beeinträchtigt, so dass diese Rotation geprüft und durch bekannte chirurgische Eingriffe an den Weichteilen in der gewünschten Weise verändert werden kann.

Ist diese Position richtig, kann mit Hilfe der Skala an der Führungsschiene der Messschlitten mit dem Schneidblock in die richtige Position zur Osteotomie der distalen Kondylen gebracht werden. Dabei ist der Abstand der Schnitte der Tibia und am Femur der einzusetzenden Prothese angepasst. Das erfindungsgemässe Bezugssystem ist nicht nur für die Osteotomien sondern ebenfalls beim Einsetzen der Prothesenkomponenten wichtig, da ihre korrekte Anpassung, bzw. die richtigen Achsenverhältnisse jederzeit während dieser entscheidenden Operationsphase kontrolliert werden können.

Die laterale Anordnung des Bezugssystem verringert Parallaxenfehler bei der Kontrolle der Achsenstellung. Solche Parallaxenfehler sind bei frontalen Richtstäben von herkömmlichen Instrumentarien grösser, da die Neutralrotationslage des gestreckten Beines wegen der Abdeckung mit sterilen Tüchern intraoperativ nicht zuverlässig hergestellt werden kann.

Die korrekte Beinachsenstellung bei gestrecktem Kniegelenk wird mit dem Zielstab kontrolliert. Hierzu wird präoperativ auf einer Beckenübersichtsaufnahme die Distanz von Spina zum Hüftgelenkzentrum gemessen und am distalen Ende des Instrumentariums auf das Zielelement bzw. den Spinazeiger übertragen.

Das erfindungsgemässe Bezugssystem eignet sich grundsätzlich für eine beliebige Reihenfolge der Osteotomien. Diese Reihenfolge wird vom operierenden Chirurgen je nach dem Zustand des Kniegelenkes bzw. der vorliegenden Achsenstellung festgelegt.

Im folgenden wird die Erfindung beispielsweise anhand der beiliegenden Zeichnungen näher erläutert. Es zeigen:

Fig. 1 eine perspektivische Uebersichtszeichnung eines erfindungsgemässen Bezugssystems und der Knochenteile, auf welche es angewendet wird,

Fig. 2 das 90° flektierte, mit Hilfe eines Spanninstrumentes distrahierte Kniegelenk, auf welches das erfindungsgemässe Bezugssystem angewendet wird,

Fig. 3 einen Tastbügel in perspektivischer Darstellung,

Fig. 4 das flektierte Kniegelenk mit Schneidblock und Tastbügel des erfindungsgemässen Bezugssystems, wobei die durchzuführenden Osteotomien sichtbar sind,

Fig. 5 eine Seitenansicht des erfindungsgemässen Bezugssystem

Fig. 6 eine Vorderansicht des erfindungsgemässen Bezugssystem

Fig. 7 Explosionszeichnung eines Spanninstrumentes für das gestreckte Knie,

Fig. 8 eine Seitenansicht des Bezugssystems, wobei die Wirkung des ersten Spanninstrumentes am gestreckten Knie zur Geltung kommt,

Fig. 9 eine Vorderansicht des Spanninstrumentes für das gestreckte Knie.

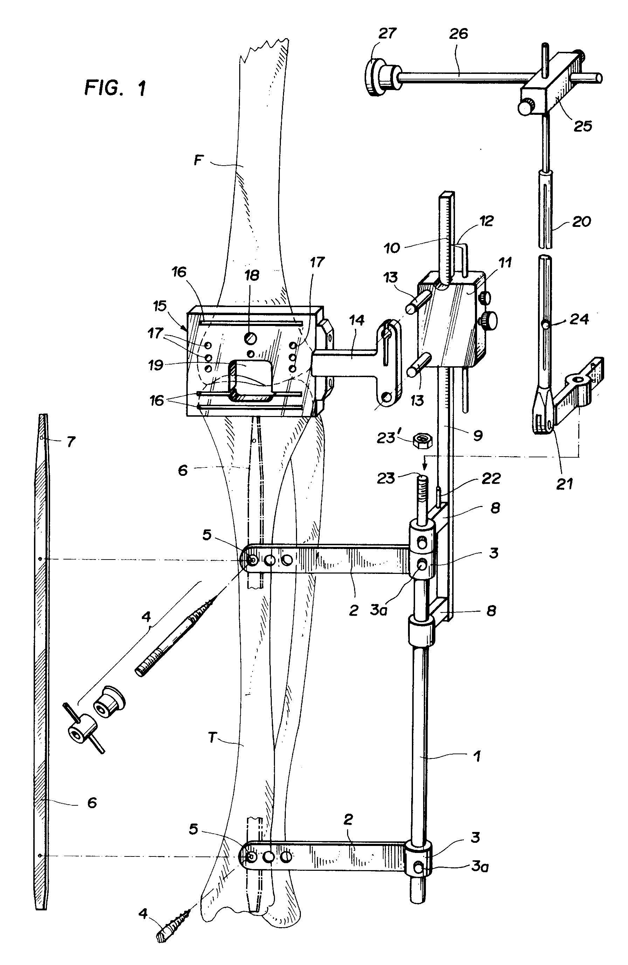

In Fig. 1 ist eine Uebersicht des Instrumentariums dargestellt, weiches die wesentlichen Bestandteile des erfindungsgemässen Bezugssystems umfasst. Es sind ebenfalls die Knochen Tibia T und Femur F dargestellt, auf welche das Bezugssystem zur Anwendung kommt. Am Messstab 1 sind die lateralen Befestigungsarme 2 angeordnet, welche mittels den Befestigungsmitteln 3, 3a mit dem Messstab verbunden sind. Der Messstab wird mittels der Schanzschrauben 4 durch die Löcher 5 in den Befestigungsarmen 2 an der Tibia T befestigt. Die zugehörigen Bohrungen 5 für die modifizierten Schanzschrauben werden im Zuge der Operationsvorbereitung mit der Lehre 6 in die Tibia gebohrt, wobei die Bohrlehre mit einem durch die Bohrung 7 in den Tibiakopf eingeschlagenen Steinmann-Nagel am Tibiakopf fixiert wird.

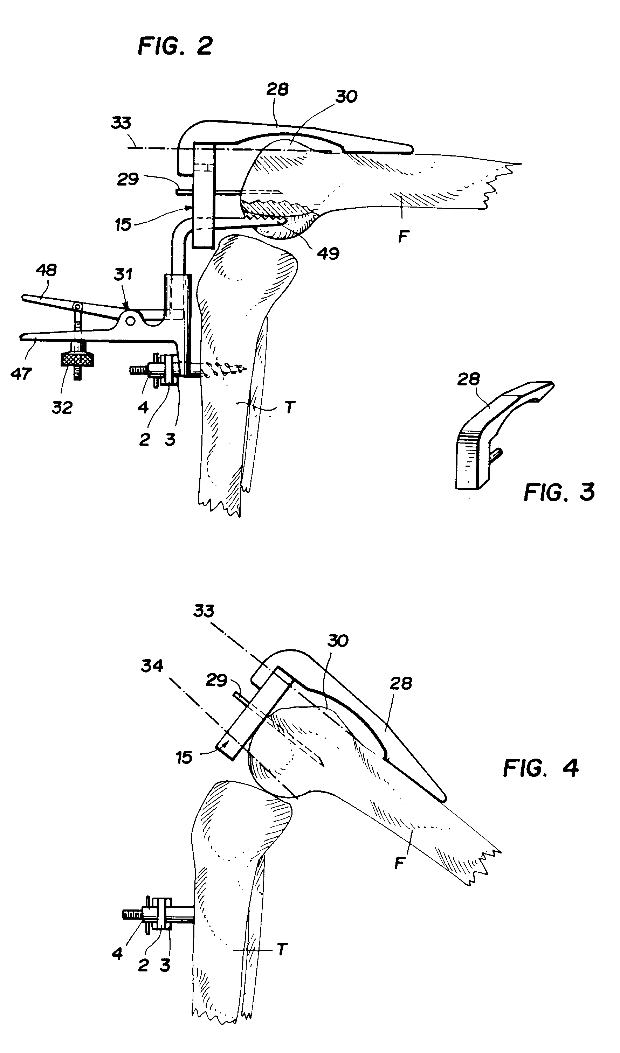

Am Messstab 1 ist mittels den Befestigungsmitteln 8 eine Führungsschiene 9 befestigt. Auf dieser Führungsschiene ist die Skala 10 sichtbar. Auf der Führungsschiene 9 befindet sich ein Messschlitten 11, welcher im Bereich der Skala 10 in die für die Osteotomien erforderlichen Lage verschoben werden kann. Alternativ könnte der Messstab 1 und die Führungsschiene 9 aus einem Stück bestehen. Im vorliegenden Beispiel ist die Führungsschiene 9 bezüglich der Frontalebene zurückversetzt, damit das Operationsfeld optimal freigegeben wird. Am Messschlitten ist ein beweglicher Zeiger 12 angeordnet, mit welchem die Eichung der Skala für jede Operation vorgenommen werden kann. Der Messschlitten weist Führungsstäbe 13 auf, welche zur Führung des Führungselementes 14 des Schneidblockes 15 dienen. Auf diesen Führungsstäben 13 kann der Schneidblock 15 fixierbar nach oben und unten verschoben werden. Der Schneidblock 15 weist Schlitze 16 auf, welche zur Einführung eines Sägeinstrumentes dienen und besitzt verschiedene Löcher 17 zur Aufnahme von Steinmann-Nägeln. Das Loch 18 dient zur Befestigung des Tastbügels gemäss Fig. 3, welcher bei Osteotomien am Femur in flektierter Lage des Knies zur Anwendung kommt. Die Oeffnung 19 dient zum Einführen eines Spanninstrumentes für Osteotomien am flektierten Knie (vgl. Fig. 2). Am Messstab 1 ist ein Zielstab 20 abnehmbar angeordnet. In der vorliegenden Darstellung ist dieser im abgenommenen Zustand dargestellt. Der Zielstab 20 wird mittels des Scharnierteils 21 am Dorn 22 und an einer Schraubverbindung 23 befestigt. Während der Operation kann der Zielstab 20 weggeklappt werden, indem er um 180° um das Scharnier gedreht wird, wobei ein unbehinderter Zugang zum Operationsfeld erreicht wird. Zur besseren Handhabung ist der Zielstab 20 in der vorliegenden Ausführungsform teleskopartig zusammenschiebbar, wobei der in eine Nut eingreifende Stift 24 eine Verdrehsicherung bewirkt. Mittels einer abnehmbaren Halterung 25 ist das Zielelement 26 verschiebbar angebracht. Es kann durch Anziehen der Schraube an der Halterung 25 fixiert werden. Das Zielelement 26 läuft an seinem Ende in ein knopfartiges Gebilde 27 aus, welches auf den Bezugspunkt der Operation, nämlich einer Schraube B in der Spina anterior superior des Beckens ausgerichtet werden kann.

In den Fig. 2 und 3 ist die Position des Schneidblockkes 15 mit aufgesetztem Tastbügel 28 bezüglich des flektierten Knies ersichtlich. Der Schneidblock ist mit zwei Steinmann-Nägeln 29 am Femur F befestigt. Mittels des obersten Sägeschlitzes werden die ventralen Kondylen 30 osteotomiert. Dabei wird das Femur mittels eines Spanninstrumentes 31 von der Tibia T weggedrückt. Der Arm 49 des Spanninstrumentes 31 für das flektierte Knie wird durch Fenster 19 im Schneidblock 15 eingesetzt. Der Arm des Teils 47 des Spanninstrumentes 31 wird auf der oberen modifizierten Schanzschraube 4 an der Tibia T abgestützt. Die Schraube 32 dient zur Feststellung der Teile 47 und 48. Im weiteren ist die Osteotomie 33 der ventralen Femurkondylen angedeutet.

In Fig. 3 ist der Tastbügel 28 in perspektivischer Darstellung abgebildet.

In Fig. 4 ist insbesondere auch die Schneidlinie 34 ersichtlich, welche die Lage der Osteotomie der dorsalen Kondylen anzeigt.

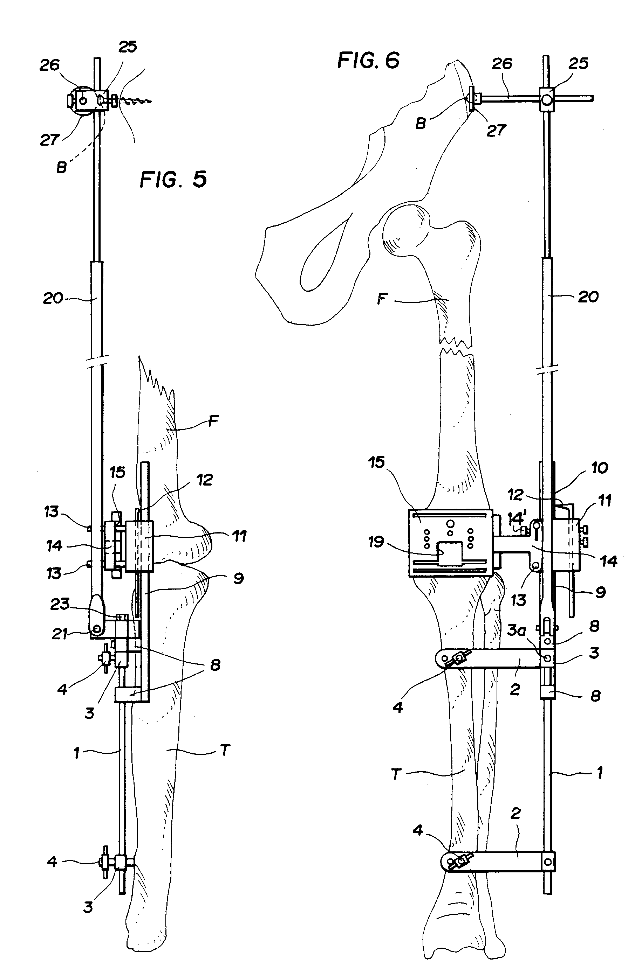

Fig. 5 zeigt eine Seitenansicht des erfindungsgemässen Bezugssystem bei gestrecktem Knie. Hier ist die Befestigung des Messstabes 1 durch die Schanzschrauben 4 an der Tibia T ersichtlich. Im vorliegenden Beispiel ist die Führungsschiene 9, auf welcher der Messschlitten 11 geführt wird, bezüglich des an der Tibia befestigten Teils des Messstabes 1 zurückversetzt. Dadurch wird eine bessere Zugänglichkeit des Operationsfeldes bewirkt. Am zurückversetzten Teil des Messstabes ist der Messschlitten 11 angebracht, welcher den beweglichen Zeiger 12 zur Ablesung der Skala besitzt. Am Schlitten sind die Führungsstäbe 13 befestigt, auf welchen der Schneidblock 15 mittels dem Führungselement 14 beweglich angebracht ist. Am Messstab 1 ist mittels des Dornes 22 und der Schraubverbindung 23 der Zielstab 20 befestigt, welcher mittels des Scharnierteils 21 zurückgeklappt werden kann. Erforderlichenfalls kann der Zielstab 20 durch Lösen der Mutter 23' an der Schraubverbindung 23 entfernt werden. Am oberen Ende des Zielstabes ist die Halterung 25 ersichtlich, mittels welcher - das Zielelement 26 mit dem Tastelement 27 in die richtige Lage gebracht werden kann.

Die Fig. 6 zeigt die gleiche Anordnung, jedoch in Vorderansicht. Hier ist die laterale Befestigung des Messstabes 1 mittels der Befestigungsarme 2, die durch die Schanzschrauben 4 an der Tibia befestigt sind, deutlich ersichtlich. Es wird ebenfalls die Lage des Schneidblockes 15 vor dem Knie verdeutlicht. Die Führungsschiene 9, auf welcher der Messschlitten 11 läuft, ist so angeordnet, dass der Schneidblock 15 für sämtliche erforderlichen Schnitte in die richtige Position gebracht werden kann. Weiter kann die Funktion des Zielstabes 20 mit dem Zielelement 26 und dem Tastelement 27 abgeleitet werden. Ist vor der Operation die Lage des Zielelementes 26 am Zielstab 20 und der Abstand des Tastelementes 27 vom Zielstab 20 bestimmt, kann durch Anlegen des Tastelementes 27 an eine Schraube in der Spina iliaca anterior superior als Bezugspunkt die Lage der Tibia jederzeit während der Operation korrigiert werden.

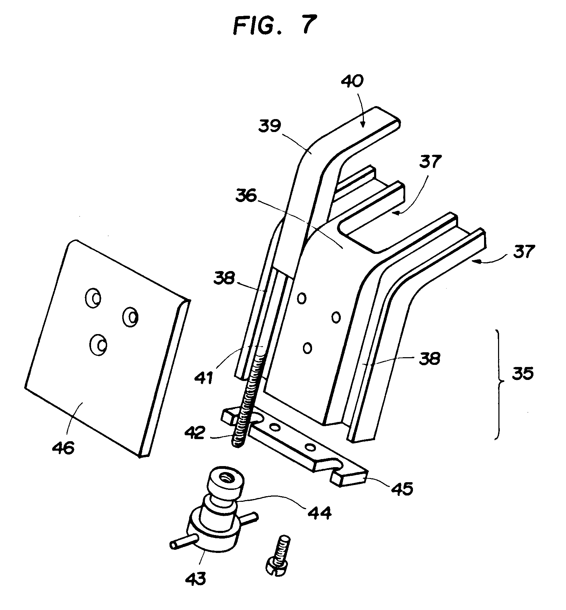

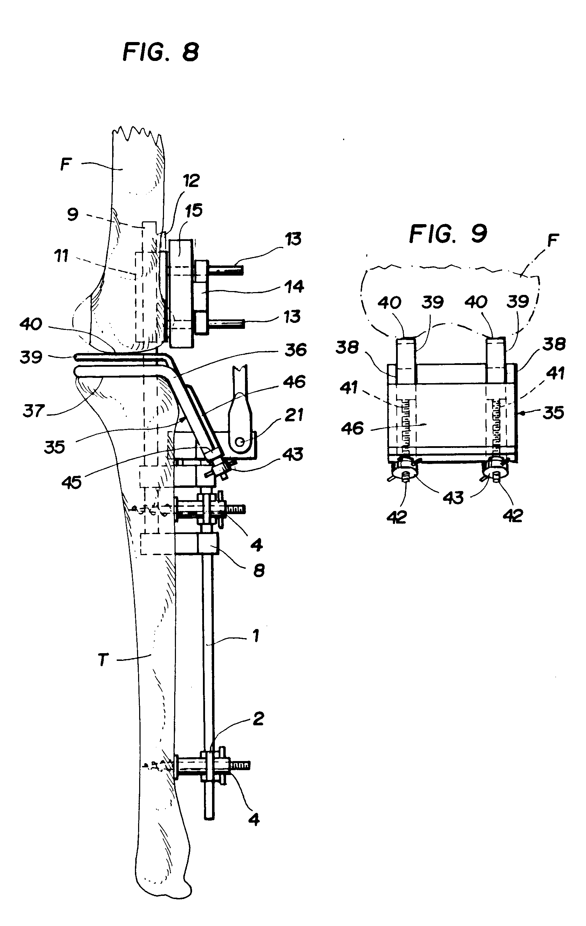

Fig. 7 zeigt ein Spanninstrument 35, deren Anwendung durch Fig. 8 näher erläutert wird. Der Teil 36 weist Auflageflächen 37 für die osteotomierte Tibia auf. In den Führungsnuten 38 sind die Teile 39 vorhanden, welche Auflageflächen 40 für die Femurkondylen aufweisen. An den Teilen 39 ist je ein Dorn 41 angeordnet, an dessen Ende ein Gewinde 42 vorhanden ist. Auf dieses Gewinde passt die Mutter 43 mit der radialen Nut 44. Mittels der Platte 45 und der Deckplatte 46 werden die Teile 39 und die Muttern 43 auf dem Teil 36 zusammengehalten. Durch diese Anordnung können durch Drehen der Muttern 43 die Teile 39 bezüglich dem Teil 36 präzise bewegt werden. Die Auflageflächen 37 und 40 bleiben dabei parallel; einzig der Abstand erfährt eine Aenderung.

In Fig. 8 ist die Anwendung des Spanninstrumentes 35 im Zusammenhang mit dem erfindungsgemässen Bezugssystem dargestellt. Die Darstellung des Bezugssystems entspricht im wesentlichen derjenigen von Fig. 5. Im Gegensatz dazu ist hier die Osteotomie der Tibia und der dorsalen und ventralen Femurkondylen bereits durchgeführt. Die Lage des Instrumentariums entspricht der Vorbereitung für die Osteotomie der distalen Femurkondylen. Durch den Druck der Auflageflächen 37, 40 wird die Tibia vom Femur weggedrückt. Ist die korrekte Position eingestellt, kann der Schneidblock in die vorbestimmte Position gebracht und die Osteotomie durchgeführt werden.

Fig. 9 zeigt das Spanninstrument 35 von vorne. Dabei ist insbesondere ersichtlich, wie die Auflageflächen 40 der beweglichen Teile 39 auf die Femurkondylen einwirken. Es ist ersichtlich, dass durch Drehen der mit der Nut versehenen Muttern 43 der Abstand von Tibia und Femur vergrössert oder verkleinert werden kann. Der nach unten gerichtete Teil 36 mit der Deckplatte 46 bewirkt eine optimale Zugänglichkeit des Operationsfeldes für die Durchführung von Osteotomien am Femur.

Ein Vorteil des erfindungsgemässen Instrumentariums besteht darin, dass die Reihenfolge der Osteotomien dem Chirurgen freisteht. Bei einem Gelenk mit erheblicher fixierter frontaler Fehlstellung wird er zuerst die Osteotomie der proximalen Tibia vornehmen und anschliessend ein Weichteil-Release durchführen. Die Osteotomie der ventralen und dorsalen Femurkondylen nach der Korrektur der Ligamente, gewährleistet dann ein stabiles Gelenk, sowohl in Extension als auch in Flexion. Bei Gelenken mit weitgehend korrekter Achsenstellung kann mit der Osteotomie der ventralen und dorsalen Femurkondylen begonnen werden.

Bei der Verwendung des Schneidblockes in der flektierten Stellung des Knies, wird dessen Lage durch den Tastbügel und durch die Steinmann-Nägel bestimmt. Die Osteotomie der ventralen Kondylen erfolgt durch den obersten Sägeschlitz und diejenige der dorsalen Kondylen durch den mittleren Sägeschlitz. Der Abstand der Bohrungen im Schneidblock für die Fixierung durch die Steinmann-Nägel entspricht den verschiedenen Prothesengrössen. Der Schneidblock kann abgehoben und um den Bohrungsabstand verschoben wieder auf die zwei Steinmann-Nägel aufgesteckt werden.

Durch die Möglichkeit, dass der Schneidblock zusätzlich rechtwinklig zum Messstab auf Führungsstäben des Messschlittens in sagitaler Richtung verschiebbar ist, kann er für jede Osteotomie an Tibia und Femur in die optimale Position gebracht werden.

Die Lage des Schneidblockes kann entsprechend der Komponentengrössen der Prothesen einer dem Instrumentarium beigegebenen Tabelle abgelesen werden.

Wie schon erwähnt, wird die korrekte Beinachsenstellung bei gestrecktem Kniegelenk mit dem Zielstab kontrolliert. Präoperativ wird auf einer Bekkenübersichtsaufnahme die Distanz zwischen Spina iliaca anterior superior zum Hüftgelenkszentrum gemessen und am distalen Ende des Zielstabes mittels dem Tastelement oder Spinazeiger übertragen.

Die richtige Resektionshöhe wird am Messstab eingestellt, der Schneidblock wird auf die osteotomierten ventralen Kondylen mit einer Corticalisschraube mit einem Durchmesser von 4,5 mm aufgeschraubt. Die distalen Kondylen werden durch den untersten Schlitz des Schneidblockes osteotomiert, wobei die korrekte Bandspannung mit Hilfe des Spanners hergestellt wird und die Achsenstellung mit dem Zielstab wiederholt kontrolliert wird.

**Patentansprüche**

1. Der Implantation von kondylären Knie-Totalprothesen unter Verwendung von Spannvorrichtungen für die das Knie umgebenden Strukturen dienendes Bezugssystem, welches sich auf die Tibia bezieht, wobei es ein Zielelement (26, 27) zur Ausrichtung der Beinachse umfasst, welches auf einen Bezugspunkt am Bekkenknochen gerichtet wird, gekennzeichnet durch einen Messstab (1) mit mindestens zwei Befestigungsarmen (2) zu dessen Befestigung lateral an der Tibia (T), parallel zur Tibialängsachse, wobei eine Führungsschiene (9) des Messstabes (1) einen fixierbaren Messschlitten (11) aufweist, welcher in Längsrichtung verschoben werden kann, dessen Position in der Längsrichtung mittels einer Längenmassskala (10) neben dem zu operierenden Knie genau festlegbar ist, seitlich am Messschlitten ein entfernbarer Schneidblock (15) als Lehre für die Osteotomien in solcher Weise angeordnet ist, dass er vor dem Knie in Position gebracht werden kann, auf am Messschlitten (11) befindlichen Führungsstäben (13) rechtwinklig zur Frontalebene und auf der Führungsschiene (9) parallel zur Tibialängsachse beweglich ist und in der vorbestimmten Position fixiert werden kann, am Messstab ein Zielstab (20) befestigt ist, welcher die Verlängerung des Messstabes (1) in Richtung des Beckens bildet und eine solche Länge aufweist, dass sein Ende bis zum Becken reicht, wobei am oberen Ende am Zielstab ein bewegliches Zielelement (26, 27) angeordnet ist, welches zur Einstellung entlang des Zielstabes und im rechten Winkel zur Sagittalebene bewegt und fixiert werden kann, damit mittels eines bestimmten Bezugspunktes (B) am Beckenknochen die richtige Position des Unterschenkels (T) während der Operation eingestellt oder kontrolliert werden kann.

2. Bezugssystem gemäss Anspruch 1, dadurch gekennzeichnet, dass zwei laterale Befestigungsarme (2) mit einer Länge von 8 - 15 cm und Befestigungsmittel (3, 3a) am Messstab vorhanden sind, welche um den Messstab (1) als Achse um 180° zur Anwendung am linken oder rechten Bein drehbar sind und in der richtigen Position mittels Befestigungsschrauben (3a) fixiert werden können und die Befestigungsarme (2) auf der dem Messstab abgewandeten Seite mehrere Befestigungsbohrungen (5) für Knochenschrauben (4) aufweisen, damit der Abstand des Messstabes (1) von der Tibia (T) derart eingestellt werden kann, dass der Schneidblock mittig vor der Fossa intercondylica liegt.

3. Bezugssystem gemäss Anspruch 1 oder 2, dadurch gekennzeichnet, dass der Zielstab (20) abnehmbar und gegenüber der Führungsschiene (9) verdrehsicher befestigt ist und mit einem Scharnier versehen ist, damit er in der Sagittalebene schwenkbar ist, damit das Operationsfeld nötigenfalls besser zugänglich gemacht werden kann.

4. Bezugssystem gemäss einem der Ansprüche 1 - 3, dadurch gekennzeichnet, dass der Messschlitten (11) zur Eichung des Bezugssystems einen verstellbaren Zeiger (12) aufweist.

5. Bezugssystem gemäss einem der Ansprüche 1 - 4, dadurch gekennzeichnet, dass der Messschlitten (11) mindestens zwei Führungsstäbe (13) aufweist, welche rechtwinklig zur Frontalebene angeordnet sind und der Schneidblock (15) ein entsprechendes Führungselement (14) aufweist, damit er längs der Führungsstäbe (13) verschoben und in der gewünschten Position fixiert werden kann.

6. Bezugssystem gemäss einem der Ansprüche 1 - 5, dadurch gekennzeichnet, dass der Schneidblock (15) mehrere Schlitze (16) aufweist, welche als Lehre für ein Sägegeräte zur Durchführung von Osteotomien dienen.

7. Bezugssystem gemäss einem der Ansprüche 1 - 6, dadurch gekennzeichnet, dass der Schneidblock (15) eine Oeffnung (19) aufweist, die so dimensioniert ist, dass durch sie hindurch ein Spanninstrument (31) eingeführt werden kann, so dass dieses Spanninstrument ohne Entfernung des Schneidblockes am flektierten Knie angewendet werden kann.

8. Bezugssystem gemäss einem der Ansprüche 1 - 7, dadurch gekennzeichnet, dass am Schneidblock (15) ein abnehmbarer Tastbügel (28) vorhanden ist, welcher zur Positionierung des Schneidblockes (15) für die Definition des O-Punktes als Bezugspunkt für die Osteotomien und für die Osteotomien der ventralen und dorsalen Femurkondylen dient.

9. Bezugssystem gemäss einem der Ansprüch 1 - 8, dadurch gekennzeichnet, dass das Zielelement (26) als Stab ausgebildet ist, der an seinem Ende ein knopfförmiges Tastelement (27) zum Anlegen an die Spina iliaca anterior superior aufweist und mit einer lösbaren Halterung (25) am Zielstab befestigt ist.

10. Bezugssystem gemäss Anspruch 1 - 9, dadurch gekennzeichnet, dass es ein Spanninstrument (31) zum Spannen der Kniebänder beim flektierten Kniegelenk zur Erzeugung einer Kraft zwischen dem Femur und der Tibia im Zuge einer Operation unter Verwendung des Bezugssystems umfasst, welches Spanninstrument zangenartig ausgebildet ist, wobei es zwei durch eine Achse verbundene bewegliche Teile (47, 48) aufweist, die durch ein Mittel in der gewünschten Position präzise reguliert und blockiert werden können, wobei der gebogene Arm (49) des Teils (48) am Dach der Fossa intercondylica zur Ausübung einer Kraft auf das Femur angesetzt werden kann, und der Teil (47) ebenfalls einen Arm aufweist, der jedoch so ausgebildet ist, dass er auf der oberen Knochenschraube (4), die bereits zur Befestigung des lateralen Arms (2) eingedreht worden ist, abgestützt werden kann, wobei die Gegenkraft durch die Knochenschraube (4) aufgenommen werden muss.

11. Bezugssystem gemäss einem der Ansprüche 1 - 9, dadurch gekennzeichnet, dass es eine Bohrlehre (6) zum Anbringen von Bohrungen in der Tibia zur Befestigung des Bezugssystems umfasst, welche Bohrlehre aus einem flachen Stab besteht, welcher an der richtigen Position Löcher für die Befestigung der Halterungsarme (2) des Messstabes (1) besitzt und überdies ein weiteres Loch (7) aufweist, mit welchem die Bohrlehre durch einen Nagel an der Tibia provisorisch befestigt werden kann.

## Claims

1. Positioning device for the implantation of total condylar knee protheses using tension devices for the structures surrounding the knee, which relates to the tibia, comprising a sighting element (26, 27) for alignment of the leg axis, which is aimed at a landmark on the pelvic bone, characterized by a measuring rod (1) with at least two attachment arms (2) for lateral attachment on the tibia (T), parallel to the tibia longitudinal axis, a guide rail (9) of the measuring rod (1) having a fixible measuring carriage (11), which can be slid in longitudinal direction, its position in the longitudinal direction being exactly determinable by means of a graduated scale of length (10) next to the knee to be operated, on the side of the measuring carriage a removable cutting block (15) as a gauge for the osteotomies disposed in such a way that it can be put into position in front of the knee, movable at right angles to the frontal plane on guide pins (13) located on the measuring carriage (11) and parallel to the tibia longitudinal axis on the guide rail (9), and which can be fixed in a pre-determined position, attached to the measuring rod is an alignment bar (20), which forms the extension of the measuring rod (1) in the direction of the pelvis and has a length such that its end reaches to the pelvis, on the upper end on the alignment bar a movable sighting element (26, 27) being disposed, which element can be moved and fixed along the alignment bar and at a right angle to the sagittal plane for adjustment so that the correct position of the lower leg (T) can be set or checked during the operation by means of a certain point of reference (B) on the pelvic bone.

2. Positioning device according to claim 1, characterized in that on the measuring rod two lateral attachment arms (2) with a length of 8 to 15 cm and attachment means (3, 3a) are provided, which are rotatable around the measuring rod (1) as an axis at 180 degrees for application on the left or right leg and can be fixed in the correct position by means of attachment screws (3a), and the attachment arms (2) on which several attachment holes (5) for bone screws (4) are provided on the side turned away from the measuring rod so that the distance of the measuring rod (1) from the tibia (T) can be adjusted in such a way that the cutting block lies concentrically in front of the Fossa intercondylica.

3. Positioning device according to claim 1 or 2, characterized in that the alignment bar (20) is detachable and is fixed in a way that it cannot be twisted in relation to the guide rail (9) and is provided with a hinge, so that it can be swivelled on the sagittal plane so that the operation site can be made more accessible if necessary.

4. Positioning device according to one of the claims 1 to 3, characterized in that the measuring carriage (11) has an adjustable indicator (12) for calibration of the positioning device.

5. Positioning device according to one of the claims 1 to 4, characterized in that the measuring carriage (11) has at least two guide pins (13), which are arranged at a right angle to the frontal plane and the cutting block has a corresponding guide element (14) so that it can be pushed along the guide rod (13) and can be fixed in the desired position.

6. Positioning device according to one of the claims 1 to 5, characterized in that the cutting block (15) has several slits (16), which serve as a gauge for a sawing instrument in carrying out osteotomies.

7. Positioning device according to one of the claims 1 to 6, characterized in that the cutting block (15) has an aperture (19), which is dimensioned in such a way that a tension device (31) can be introduced through it so that this tension device can be used on the flexed knee without the cutting block being removed.

8. Positioning system according to one of the claims 1 to 7, characterized in that a removable caliper bracket (28) on the cutting block (15) is provided, which serves to position the cutting block (15) to define the O point as the landmark for osteotomies and for the osteotomies of the ventral and dorsal femur condyles..

9. Positioning system according to one of the claims 1 to 8, characterized in that the sighting element (26) is formed as a rod which has a knob-shaped press element (27) for placement on the spina iliaca anterior superior on its end and is attached to the alignment bar with a detachable fixing device (25).

10. Positioning device according to claims 1 to 9, characterized in that it comprises a tension device (31) for tensioning the genicular ligaments in the flexed knee joint to generate a force between the femur and the tibia in the course of an operation using the positioning device, the said tension device of pincers-like form, having two movable parts (47, 48) connected by an axis which parts can be regulated precisely and blocked in the desired position through a means, whereby the bent arm (49) of the part (48) on the roof of the fossa intercondylica can be applied to exert a force on the femur, and the part (47) also has an arm which is formed in such a way however that it can be braced against the upper Bone screw (4), which has already been screwed in to fix the lateral arm (2), the counterforce being necessarily taken up by the bone screw (4).

11. Positioning device according to one of the claims 1 to 9, characterized in that it comprises a hole gauge (6) for putting bores in the tibia for fastening the positioning device, the said bore gauge consisting of a flat stick which has holes at the correct position for fastening the attachment arms (2) of the measuring rod (1) and has in addition a further hole (7) through which the hole gauge can be provisionally fixed to the tibia with a nail.

**Revendications**

1. Dispositif de positionnement pour l'implantation de prothèses condyliennes totales de genou par l'emploi de dispositifs de tension pour des structures entourant le genou, laquelle se rapporte au tibia, comprenant un élément de visée (26,27) pour l'alignement de l'axe de la jambe, lequel est aligné sur un point de recouvrement de l'os du bassin, caractérisé par une échelle graduée (1) avec au moins deux bras de fixation (2) pour sa fixation latérale au tibia (T), parallèle à l'axe longitudinal du tibia, un rail de guidage (9) de l'échelle graduée (1) comprenant un chariot de mesure (11) fixable, pouvant être déplacé selon la direction longitudinale, dont la position longitudinale peut être fixée exactement par une échelle de mesure de longueur (10), à côté du genou à opérer, un bloc de coupe (15) éloignable étant disposé à côté du chariot de mesure pour la conduite de l'ostéotomie, de telle manière qu'il puisse être appliqué en position devant le genou, qu'il soit déplaçable perpendiculairement à la surface frontale sur des petites tiges de guidage (13) disposées sur le chariot de mesure (11) et, parallèlement à l'axe du tibia par le rail de guidage (9), et qu'il puisse être fixé en la position prédéterminée, une tige de visée (20) étant fixée à l'échelle graduée, formant la prolongation de l'échelle de visée en direction du bassin, et étant d'une longueur telle que son extrémité atteint le bassin, un élément de visée (26,27) étant fixé de manière amovible à l'extrémité supérieure de la tige de visée, pouvant être installé le long de la tige de visée et pouvant être déplacé et fixé selon un angle droit par rapport au plan sagittal, permettant d'ajuster et de contrôler la position correcte de la partie inférieure de la jambe (T) durant l'opération au-moyen d'un point de recouvrement (B) de l'os du bassin.

2. Dispositif de positionnement selon la revendication 1, caractérisé en ce que deux bras de fixation latéraux (2) d'une longueur de 8 - 15 cm ainsi que des moyens de fixation (3,3a) à l'échelle graduée sont prévus, lesquels sont pivotables de 180° autour de l'axe de l'échelle graduée (1) pour s'adapter à la jambe gauche ou à la jambe droite, pouvant être fixés dans la position correcte par les vis de fixation (3a), les bras de fixation (2) comprenant, du côté opposé à l'échelle graduée, plusieurs trous de fixation (5) pour des vis à os (4), faisant que la distance de la règle graduée (1) au tibia (T) peut être ajustée de telle manière que le bloc de coupe soit situé au milieu de la Fossa intercondylica.

3. Dispositif de positionnement selon l'une des revendications 1 ou 2, caractérisé en ce que la tige de visée (20) est fixée de manière amovible et en face du rail de guidage (9) sans torsion, étant prévu avec une charnière permettant de la pivoter dans le plan sagittal afin que le champ opératoire soit si nécessaire mieux accessible.

4. Dispositif de positionnement selon l'une des revendications 1 à 3, caractérisé en ce que le chariot de mesure (11) comprend une aiguille (12) réglable pour l'étalonnage dudit dispositif de positionnement.

5. Dispositif de positionnement selon l'une des revendications 1 à 4, caractérisé en ce que le chariot de mesure (11) comprend au moins deux petites tiges de guidage (13) disposées perpendiculairement au plan frontal, et en ce que le bloc de coupe (15) comprend un élément de guidage (14) correspondant, pouvant être déplacé et fixé selon une position désirée le long des petites tiges de guidage (13).

6. Dispositif de positionnement selon l'une des revendications 1 à 5, caractérisé en ce que le bloc de coupe (15) comprend une pluralité de rainures (16) servant au guidage d'un dispositif de scie pour l'exécution d'ostéotomies.

7. Dispositif de positionnement selon l'une des revendications 1 à 6, caractérisé en ce que le bloc de coupe (15) comprend une ouverture (19) dimensionnée de telle manière qu'un tendeur (31) peut y être introduit, ledit tendeur pouvant être appliqué à un genou fléchi sans retrait du bloc de coupe.

8. Dispositif de positionnement selon l'une des revendications 1 à 7, caractérisé en ce qu'une pièce de touche (28) retirable est disposée sur le bloc de coupe (15), laquelle sert au positionnement du bloc de coupe pour la définition du point 0 comme point de recouvrement pour l'ostéotomie et pour l'ostéotomie des condyles fémuraux ventraux et dorsaux.

9. Dispositif de positionnement selon l'une des revendications 1 à 8, caractérisé en ce que l'élément de visée (26) est constitué d'une barre comprenant à une de ses extrémité un élément de touche (27) en forme de bouton pour se placer sur le Spina iliaca anterior superior et un dispositif de fixation (25) amovible pour le fixer à la tige de visée.

10. Dispositif de positionnement selon l'une des revendications 1 à 9, caractérisé en ce qu'il comprend un tendeur (31) pour tendre les ligaments lors d'une flexion du genou afin d'obtenir une force entre le fémur et le tibia lors d'une opération utilisant le dispositif de positionnement, ledit tendeur étant en forme de pince, comprenant deux parties (47,48) mobiles reliées par un axe, pouvant être réglées et bloquées selon une position précise désirée par un dispositif adéquat, le bras recourbé (49) de la partie (48) pouvant être disposé au sommet de la Fossa intercondylica pour exercer une force sur le fémur, la partie (47) comprenant également un bras, disposé cependant de telle manière qu'il puisse être supporté par la vis d'os supérieure (4) déjà vissée pour fixer le bras latéral (2), la force de réaction pouvant s'exercer à travers la vis d'os (4).

11. Dispositif de positionnement selon l'une des revendications 1 à 9, caractérisé en ce qu'il comprend un guide de perçage (6) pour effectuer des trous dans le tibia afin de fixer ledit dispositif de positionnement, ledit guide de perçage étant constitué d'une barre plate, lequel possède des trous disposés en des positions exactes pour la fixation des bras de fixation (2) de la règle graduée (1) et comprenant un autre trou (7) après ceux-ci par lequel le guide de perçage peut être fixé provisoirement au tibia au moyen d'une aiguille.

FIG. 1

FIG. 2

FIG. 3

FIG. 4

FIG. 5

FIG. 6

FIG. 7

FIG. 8

FIG. 9